# EUROPÄISCHE PATENTSCHRIFT

(11) **EP 4 167 737 B1**
(45) Veröffentlichungstag und Bekanntmachung des Hinweises auf die Patenterteilung: **16.04.2025**
(21) Anmeldenummer: 21734793.9
(22) Anmeldetag: 17.06.2021
(51) Int. Cl.: A01N 1/02, A61F 2/28, A61F 2/46, G07C 9/00

(54) **BEARBEITBARER FORMKÖRPER MIT KNOCHENMATERIAL**
MACHINABLE SHAPED BODY WITH BONE MATERIAL
CORPS MOULÉ USINABLE RENFERMANT UNE MATIÈRE OSSEUSE

(30) Priorität: 19.06.2020 DE 102020116275
(43) Veröffentlichungstag der Anmeldung: 26.04.2023
(73) Patentinhaber: Organical CAD/CAM GmbH, 12681 Berlin (DE)
(72) Erfinder: HUTSKY, André, 16552 Schildow (DE); MARUNDE, Carsten, 16515 Zühlsdorf (DE); KLAR, Andreas, 12683 Berlin (DE)
(74) Vertreter: Eisenführ Speiser
(86) Internationale Anmeldenummer: PCT/EP2021/066447
(87) Internationale Veröffentlichungsnummer: WO 2021/255183

(56) Entgegenhaltungen:
- WO-A1-2015/172159
- US-A1- 2007 233 272
- GERBER N ET AL: "Using rapid prototyping molds to create patient specific polymethylmethacrylate implants in cranioplasty", 2010 ANNUAL INTERNATIONAL CONFERENCE OF THE IEEE ENGINEERING IN MEDICINE AND BIOLOGY SOCIETY : (EMBC 2010) ; BUENOS AIRES, ARGENTINA, 31 AUGUST - 4 SEPTEMBER 2010, IEEE, PISCATAWAY, NJ, USA, 31 August 2010 (2010-08-31), pages 3357 - 3360, XP032109602, ISBN: 978-1-4244-4123-5, DOI: 10.1109/IEMBS.2010.5627903
- HIEU L.C. ET AL: "Design and manufacturing of cranioplasty implants by 3-axis cnc milling", TECHNOLOGY AND HEALTH CARE, vol. 10, no. 5, 18 September 2002 (2002-09-18), NL, pages 413 - 423, XP055846485, ISSN: 0928-7329, DOI: 10.3233/THC-2002-10505

## Beschreibung

### Gebiet der Erfindung

Die Erfindung betrifft eine Anordnung aus einer Mehrzahl von in einen menschlichen oder tierischen Körper einsetzbaren, natürlichen und/oder künstlichen prothetischen Elementen, wie beispielsweise autogenen, isogenen, xenogenen oder allogenen Knochenstücken, mit einem Formkörper, der ein Substrat aufweist, wobei das Substrat die prothetischen Elemente abschnittsweise oder im Wesentlichen vollständig enthält und aus einem mit einem Werkzeug bearbeitbaren Material besteht, das von einem fließfähigen Zustand, in dem das Substrat mit den prothetischen Elementen verbindbar ist oder die prothetischen Elemente in das Substrat einbringbar sind, in einen festen Zustand überführbar ist. Des Weiteren betrifft die Erfindung ein Verfahren zum Einbringen einer Mehrzahl von in einen menschlichen oder tierischen Körper einsetzbaren, natürlichen und/oder künstlichen prothetischen Elementen, wie beispielsweise autogenen, isogenen, xenogenen oder allogenen Knochenstücken, in eine derartige Anordnung. Ferner betrifft die Erfindung eine Vorrichtung zur Bearbeitung von in einen menschlichen oder tierischen Körper einsetzbaren, natürlichen und/oder künstlichen prothetischen Elementen, wie beispielsweise autogenen, isogenen, xenogenen oder allogenen Knochenstücken, unter Verwendung einer Anordnung der vorgenannten Art. Schließlich betrifft die Erfindung ein Verfahren zur Bearbeitung von in einen menschlichen oder tierischen Körper einsetzbaren, natürlichen und/oder künstlichen prothetischen Elementen, wie beispielsweise autogenen, isogenen, xenogenen oder allogenen Knochenstücken, unter Verwendung einer solchen Vorrichtung und einer Anordnung der vorgenannten Art.

### Hintergrund der Erfindung

Die Verwendung von prothetischen Elementen aus künstlichem Material wie insbesondere Metall, Keramik oder Kunststoff hat sich in der Praxis bewährt. Es gibt aber auch durchaus Situationen, in denen die Verwendung von prothetischen Elementen aus natürlichem Material wie beispielsweise autogenen, isogenen, xenogenen oder allogenen Knochenstücken von Vorteil ist, was beispielsweise dann der Fall ist, wenn rein keramische Materialien zu spröde sind, um in komplexe Geometrien übertragen und mit Zugschrauben an der Defektstelle befestigt zu werden. Denn natürliches Knochenmaterial ist nicht spröde, sondern erhält die natürliche Duktilität an der Defektstelle, wodurch Handhabung, Formgebung und auch die Fixierung wesentlich erleichtert werden.

Die Kombination aus modernen dreidimensionalen Bildgebungsverfahren und industrieller CAD/CAM-Technologie führt zu besseren Planungs- und Behandlungsmethoden bei Verwendung von Prothesen jeglicher Art im Allgemeinen und in der Mund-, Kiefer- und Gesichtschirurgie im Besonderen. Aus den Daten, die durch das Bildgebungsverfahren erzeugt werden und in der Regel eine Vielzahl von einzelnen Schnittbildern enthalten, wird mit einer geeigneten Software ein dreidimensionales Modell generiert. Das Vorkonturieren von, insbesondere allogenen, Knochenblöcken am Modell eröffnet dann die Möglichkeit, die Passgenauigkeit im Vorfeld der Operation zu maximieren und führt dadurch zu einer Verkürzung der eigentlichen Dauer des Eingriffes. Durch Einsatz geeigneter Bearbeitungsvorrichtungen wie insbesondere von CNC-Fräsmaschinen lassen sich aus dem aufbereiteten Rohmaterial geometrisch anspruchsvolle, individuelle Prothesen und Transplantate herstellen. Dies gilt insbesondere auch für Dentalimplantate. Dabei empfiehlt es sich bei Verwendung von natürlichem Knochenmaterial, möglichst alle kortikalen Knochenanteile zu entfernen. Die fertiggestellten prothetischen Elemente werden nach ihrer Bearbeitung verpackt und sterilisiert.

US 2007/0233272 A1, die den nächstkommenden Stand der Technik bildet, von der die vorliegende Erfindung ausgeht, offenbart eine Anordnung aus einer Mehrzahl von in einen menschlichen oder tierischen Körper einsetzbaren, natürlichen und/oder künstlichen prothetischen Elementen in Form verstärkender Strukturelemente, mit einem Formkörper, der ein Substrat aufweist, wobei das Substrat die prothetischen Elemente abschnittsweise oder im Wesentlichen vollständig enthält. Das Substrat besteht aus einem mit einem Werkzeug bearbeitbaren Material, das ein biokompatibles Bindemittel aufweist und von einem fließfähigen Zustand, in dem die prothetischen Elemente in das Substrat einbringbar sind, durch einen Abbindeprozess in einen festen Zustand überführbar ist. Das Bindemittel weist Zement auf und enthält alloplastische, autogene, isogene, allogene oder xenogene Knochenersatzstoffe.

### Zusammenfassung der Erfindung

Es ist nun eine Aufgabe der vorliegenden Erfindung, eine einfache und zugleich optimale Lösung zur Aufbewahrung von derartigen prothetischen Elementen und zu deren Verarbeitung anzugeben.

Gelöst wird diese Aufgabe gemäß einem ersten Aspekt der vorliegenden Erfindung mit einer Anordnung aus einer Mehrzahl von in einen menschlichen oder tierischen Körper einsetzbaren, natürlichen und/oder künstlichen prothetischen Elementen, wie beispielsweise autogenen, isogenen, xenogenen oder allogenen Knochenstücken, mit einem Formkörper, der ein Substrat aufweist, wobei das Substrat die prothetischen Elemente abschnittsweise oder im Wesentlichen vollständig enthält und aus einem mit einem Werkzeug bearbeitbaren Material besteht, wobei das Material des Substrates von einem fließfähigen Zustand, in dem das Substrat mit den prothetischen Elementen verbindbar ist oder die prothetischen Elemente in das Substrat einbringbar sind, in einen festen Zustand überführbar ist, dadurch gekennzeichnet, dass das Material des Substrates sich im fließfähigen Zustand befindet, wenn die auf das Material einwirkende Temperatur einen ersten Temperaturwert hat, und einen festen Zustand einnimmt, wenn die auf das Material des Substrates einwirkende Temperatur einen unterhalb des ersten Temperaturwertes liegenden zweiten Temperaturwert hat.

Demnach wird erfindungsgemäß vorgeschlagen, einen bearbeitbaren Formkörper mit prothetischen Elementen zur Verfügung zu stellen, die mithilfe eines Substrates miteinander verbunden sind, um den Formkörper zu bilden. Unter den Begriff "prothetische Elemente" im Sinne der vorliegenden Erfindung fallen jegliche Arten von Rohlingen, Materialblöcken, vorgefertigten Halbzeugen und/oder auch für den Einsatz in menschlichen oder tierischen Körper bereits im Wesentlichen fertiggestellten Prothesen oder Transplantate, zu denen auch u.a. Dentalimplantate zählen. Die prothetischen Elemente weisen künstliches Material und/oder natürliches Material, wie beispielsweise autogene, isogene, xenogene oder allogene Knochenstücke, auf oder bestehen aus derartigen Materialien. Bevorzugt handelt es sich bei den prothetischen Elementen um Halbzeuge, um daraus Prothesen oder Transplantate herzustellen, die dann in einen menschlichen oder tierischen Körper eingesetzt werden. Dadurch, dass erfindungsgemäß die prothetischen Elemente mithilfe des Substrates in einem Formkörper zusammengefasst werden, wird eine einfache und zugleich optimale Möglichkeit zur Aufbewahrung bzw. Archivierung der prothetischen Elemente geschaffen. Außerdem ermöglicht die erfindungsgemäße Anordnung nicht nur eine einfache Lagerung einer Mehrzahl von prothetischen Elementen in einer gemeinsamen Anordnung, sondern auch deren einfachen Transport.

Da das erfindungsgemäß verwendete Substrat, durch das die einzelnen prothetischen Elemente zur Bildung des Formkörpers miteinander verbunden sind, aus einem mit einem Werkzeug bearbeitbaren Material besteht, lassen sich mithilfe einer Bearbeitungsvorrichtung die prothetischen Elemente zur Bildung von Transplantaten und Prothesen und für deren späteren Einsatz in einem menschlichen oder tierischen Körper auf einfache Weise aus dem Formkörper herausarbeiten bzw. heraustrennen. Hierzu lässt sich der erfindungsgemäße Formkörper als Fixierhilfe zur Befestigung bzw. zum Einspannen der Anordnung der prothetischen Elemente an der Halteeinrichtung einer Bearbeitungsvorrichtung verwenden und kann dabei eine Art Sicherheitspuffer bilden, wodurch einerseits eine Beschädigung der Halteeinrichtung durch das Werkzeug der Bearbeitungsvorrichtung und andererseits eine räumliche Einschränkung beim Herausarbeiten eines gewünschten prothetischen Elementes aus dem Formkörper vermieden wird. Die Handhabung ist besonders einfach, indem das Material des Substrates von einem fließfähigen Zustand, in dem das Substrat mit den prothetischen Elementen verbindbar ist oder die prothetischen Elemente in das Substrat einbringbar sind, in einen festen Zustand überführbar ist.

Schließlich zeichnet sich die erfindungsgemäße Anordnung dadurch aus, dass das Material des Substrates sich im fließfähigen Zustand befindet, wenn die auf das Material einwirkende Temperatur einen ersten Temperaturwert hat, und einen festen Zustand einnimmt, wenn die auf das Material des Substrates einwirkende Temperatur einen unterhalb des ersten Temperaturwertes liegenden zweiten Temperaturwert hat. Demnach erfolgt erfindungsgemäß die Verbindung der prothetischen Elemente zur Bildung des Formkörpers nicht über ein vom Substrat gebildetes Bindemittel mit Hilfe eines Abbindeprozesses, sondern durch Änderung des Aggregatszustandes des Substrates von "fließfähig" in "fest".

Bevorzugte Ausführungsbeispiele und Weiterbildungen der Anordnung gemäß dem ersten Aspekt der Erfindung sind in den abhängigen Ansprüchen 2 bis 5 angegeben.

Vorzugsweise liegt der erste Temperaturwert, bei dem das Substrat fließfähig ist, oberhalb der Zimmertemperatur und entspricht der zweite Temperaturwert, bei dem sich das Substrat im festen Zustand befindet, im Wesentlichen Zimmertemperatur.

Alternativ kann das Substrat aber auch aus einem solchen Material bestehen, dass der erste Temperaturwert im Wesentlichen Zimmertemperatur entspricht und der zweite Temperaturwert unterhalb der Zimmertemperatur liegt. Vorzugsweise liegt der zweite Temperaturwert unter 0°C, bei dem das Substrat einfriert und dadurch der Formkörper eine Art Eisblock bildet, sodass in diesem Fall zweckmäßigerweise das Material des Substrates Wasser oder eine isotonische Kochsalzlösung ist.

Gemäß einem weiteren bevorzugten Ausführungsbeispiel ist eine Temperaturhalteeinrichtung vorgesehen, die ausgebildet ist, den Formkörper auf den zweiten Temperaturwert zu halten, und bevorzugt Kühlmittel aufweist.

Die zuvor genannte Aufgabe wird des Weiteren gemäß einem zweiten Aspekt der vorliegenden Erfindung gelöst mit einem Verfahren zum Einbringen einer Mehrzahl von in einen menschlichen oder tierischen Körper einsetzbaren, natürlichen und/oder künstlichen prothetischen Elementen, wie beispielsweise autogenen, isogenen, xenogenen oder allogenen Knochenstücken, in eine Anordnung gemäß dem ersten Aspekt, die einen Formkörper und ein Substrat aufweist, wobei das Substrat dazu eingerichtet ist, die prothetischen Elemente abschnittsweise oder im Wesentlichen vollständig zu enthalten, dadurch gekennzeichnet, dass
I) die auf das Material des Substrates (4a) einwirkende Temperatur auf einen ersten Temperaturwert gebracht wird, bei dem sich das Material im fließfähigen Zustand befindet,
II) die prothetischen Elemente (2, 12) in das Substrat (4a) eingebracht werden und
III) anschließend die auf das Material des Substrates (4a) einwirkende Temperatur auf einen unterhalb des ersten Temperaturwertes liegenden zweiten Temperaturwert gebracht wird, bei dem das Material einen festen Zustand einnimmt,
wobei bevorzugt im Schritt II die prothetischen Elemente (2, 12) in eine Form gelegt werden und anschließend das Substrat (4a) im fließfähigen Zustand in die Form eingefüllt wird.

Des Weiteren wird die zuvor angegebene Aufgabe gemäß einem dritten Aspekt der vorliegenden Erfindung gelöst mit einer Vorrichtung zur Bearbeitung von in einen menschlichen oder tierischen Körper einsetzbaren, natürlichen und/oder künstlichen prothetischen Elementen, wie beispielsweise autogenen, isogenen, xenogenen oder allogenen Knochenstücken, unter Verwendung einer Anordnung gemäß dem ersten Aspekt, wobei die Vorrichtung eine Erfassungseinrichtung zur Erfassung der Position der prothetischen Elemente innerhalb des Formkörpers und eine Bearbeitungsvorrichtung aufweist, und wobei die Bearbeitungsvorrichtung ein Werkzeug zum abschnittsweisen oder vollständigen Heraustrennen von prothetischen Elementen aus dem Formkörper aufweist.

Mithilfe der Erfassungseinrichtung lassen sich die prothetischen Elemente innerhalb des Formkörpers genau identifizieren. Unter Berücksichtigung dieser Identifizierung ist dann die Bearbeitungseinrichtung in der Lage, mithilfe ihres Werkzeuges zumindest im Wesentlichen exakt das gewünschte prothetische Element abschnittsweise oder vollständig aus dem Formkörper herauszuarbeiten bzw. herauszutrennen, was grundsätzlich nur dann möglich ist, wenn sich der Formkörper in seinem festen Zustand befindet.

Bevorzugte Ausführungsbeispiele und Weiterbildungen der Vorrichtung gemäß dem dritten Aspekt der Erfindung sind in den abhängigen Ansprüchen 8 bis 10 angegeben.

Vorzugsweise ist die Erfassungseinrichtung zusätzlich zur Erfassung der Temperatur und/oder der Zusammensetzung und/oder der Qualität der prothetischen Elemente vorgesehen. Hierbei lässt sich mithilfe der Erfassungseinrichtung zusätzlich zur Lage auch die Qualität der im Formkörper eingeschlossenen prothetischen Elemente exakt ermitteln. Dies ist besonders vorteilhaft, da sich auf diese Weise insbesondere Abschnitte der prothetischen Elemente mit der gewünschten Qualität für einen bestimmten Abschnitt oder bestimmte Abschnitte der herzustellenden Prothese aus dem Formkörper herausarbeiten lassen. Dies gilt insbesondere für den Fall, dass die prothetischen Elemente Knochenstücke aufweisen oder aus solchen bestehen.

Aufgrund des Ergebnisses der Erfassung durch die Erfassungseinrichtung lässt sich die Herstellung von prothetischen Elementen oder Prothesen besonders präzise realisieren, da das Werkzeug der Bearbeitungseinrichtung zielgenau zu den gewünschten Abschnitten der prothetischen Elemente geführt werden kann.

Bevorzugt weist die Erfassungseinrichtung ein Röntgen-Gerät und/oder ein Computertomografie-Gerät und/oder mindestens einen Temperatursensor auf. Unter Computertomografie-Gerät in diesem Sinne wird nicht nur ein herkömmlicher Computertomograf (CT), sondern auch ein digitaler Volumentomograf (DVT) verstanden, welche dreidimensionale Röntgendaten zur Verfügung stellen, die für eine virtuelle Transplantatplanung geeignet sind.

Des Weiteren kann eine Fixiereinrichtung zum Einspannen des Formkörpers vorgesehen sein.

Zur Herstellung einer Anordnung gemäß dem ersten Aspekt durch einen Gießprozess ist vorzugsweise eine Form vorzusehen, in der die prothetischen Elemente anordenbar und in die das Substrat im fließfähigen Zustand einfüllbar ist.

Zur Herstellung einer Anordnung gemäß dem ersten Aspekt unter Verwendung eines Substrates, dessen Material bei unterschiedlichen Temperaturen seinen Aggregatzustand ändert, ist zweckmäßigerweise eine Temperaturhalteeinrichtung zum Halten der Temperatur des Formkörpers auf den gegenüber dem ersten Temperaturwert niedrigeren zweiten Temperaturwert vorzusehen, wobei vorzugsweise die Temperaturhalteeinrichtung eine Kühleinrichtung aufweist, die den Formkörper auf einen zweiten Temperaturwert unterhalb der Zimmertemperatur, vorzugsweise unter 0°C, kühlt, wodurch sich der Formkörper in eine Art Eisblock verwandelt. Schließlich wird die zuvor genannte Aufgabe gemäß einem vierten Aspekt der vorliegenden Erfindung gelöst mit einem Verfahren zur Bearbeitung von in einen menschlichen oder tierischen Körper einsetzbaren, natürlichen und/oder künstlichen prothetischen Elementen, wie beispielsweise autogenen, isogenen, xenogenen oder allogenen Knochenstücken, unter Verwendung einer Anordnung gemäß dem ersten Aspekt und einer Vorrichtung gemäß dem dritten Aspekt, wobei
A) mithilfe der Erfassungseinrichtung mindestens die Position der prothetischen Elemente innerhalb des Formkörpers ermittelt wird,
B) in Abhängigkeit von mindestens der von der Erfassungseinrichtung erfassten Position der im Formkörper enthaltenen prothetischen Elemente das Werkzeug der Bearbeitungseinrichtung in den Formkörper zu den prothetischen Elementen mit der von der Erfassungseinrichtung erfassten Position geführt wird und
C) das prothetische Element oder die prothetischen Elemente mit der von der Erfassungseinrichtung erfassten Position mithilfe des Werkzeugs der Bearbeitungseinrichtung aus dem Formkörper herausgetrennt wird bzw. werden.

Hiernach wird insbesondere der Formkörper in eine, bevorzugt bereits bestehende, Erfassungseinrichtung angeordnet oder gelegt, um die exakte Position der prothetischen Elemente sowie bedarfsweise zusätzlich die jeweilige Qualität der prothetischen Elemente und/oder im Falle der Verwendung von Knochenstücken als prothetische Elemente deren Dichte zu lokalisieren. Hierfür sollte bevorzugt der Formkörper auch mit einer exakten Nullpunktreferenzierung versehen sein, die zweckmäßigerweise definierte Marker aufweist. Anschließend können dann die Daten des so erfassten Formkörpers in eine CAM-Software eingeladen werden, um die prothetischen Elemente zumindest mit ihren herauszuarbeitenden Abschnitten innerhalb des Formkörpers an der gewünschten Position zu nesten. Denn es ist möglich, die herauszuarbeitenden Abschnitte der prothetischen Elemente oder gegebenenfalls auch die herauszuarbeitenden prothetischen Elemente insgesamt so zu nesten, dass diese bedarfsweise nur dort in den Knochen zu positionieren sind, wo sie in den menschlichen oder tierischen Körper eingesetzt werden sollen. Demnach lässt sich also eine lagerichtige Nestung in Abhängigkeit von der Knochendichte korrekt realisieren.

Bevorzugte Ausführungsbeispiele und Weiterbildungen des Verfahrens gemäß dem vierten Aspekt der Erfindung sind in den abhängigen Ansprüchen 12 und 13 angegeben.

Zweckmäßigerweise sollte vor Schritt B der Formkörper in einer Fixiereinrichtung eingespannt werden.

Eine bevorzugte Weiterbildung des genannten Verfahrens zeichnet sich dadurch aus, dass in Schritt A mithilfe der Erfassungseinrichtung des Weiteren die Dichte der prothetischen Elemente erfasst wird.

Nachfolgend wird ein bevorzugtes Ausführungsbeispiel der Erfindung anhand der beiliegenden Zeichnungen näher erläutert. Es zeigen:
- Fig. 1: schematisch im Querschnitt einen Einbettrohling, in dem beispielhaft ein Knochenhalbzeug eingebettet ist;
- Fig. 2: schematisch im Querschnitt Abschnitte einer Fixiereinrichtung einer Bearbeitungseinrichtung, in der der Einbettrohling von Fig. 1 eingespannt ist;
- Fig. 3: die gleiche Ansicht wie Fig. 2, wobei zusätzlich schematisch ein innerhalb des Knochenhalbzeuges für die Herausarbeitung bzw. Heraustrennung definiertes Knochenprodukt abgebildet ist;
- Fig. 4: schematisch in perspektivischer Draufsicht die Fixiereinrichtung der Bearbeitungseinrichtung, in der der Einbettrohling eingespannt ist, wobei in dem Einbettrohling schematisch noch zusätzlich das dort eingebettete Knochenhalbzeug mit dem darin definierten Knochenprodukt erkennbar ist;
- Fig. 5: die gleiche Ansicht wie Fig. 3, wobei Abschnitte des Einbettrohlings und des darin eingebetteten Knochenhalbzeuges zur Freilegung des herauszutrennenden Knochenproduktes bereits entfernt sind; und
- Fig. 6: eine ähnliche Ansicht wie Fig. 4, wobei jedoch zusätzlich schematisch das Knochenprodukt in einem nun im Wesentlichen vollständig vom Einbettrohling und vom Knochenhalbzeug getrennten Zustand kurz vor Herausnahme aus dem Einbettrohling abgebildet ist.

### Detaillierte Beschreibung

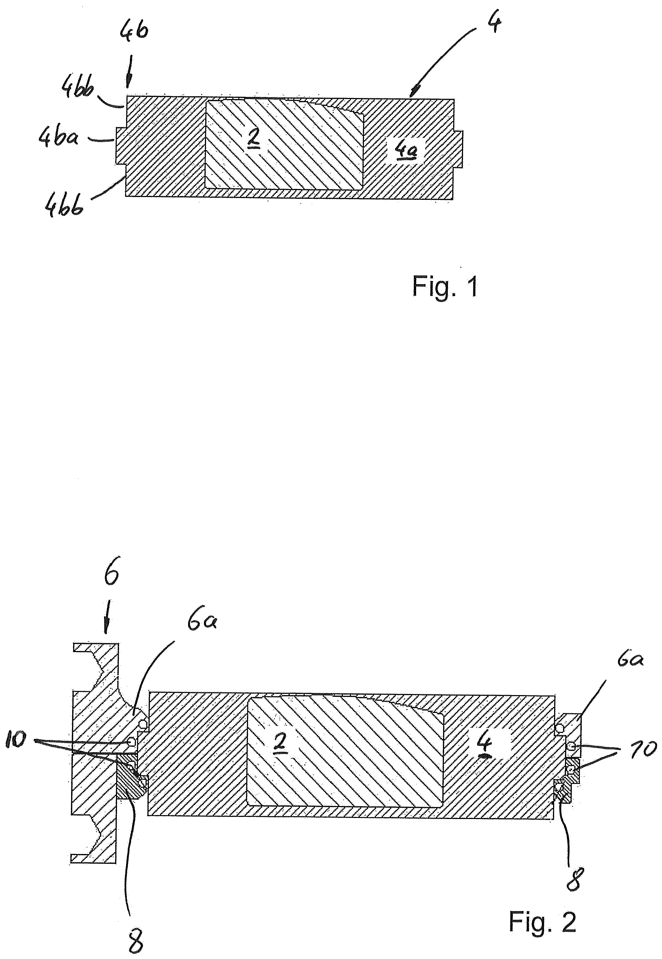

In Fig. 1 ist schematisch im Querschnitt beispielhaft ein Knochenhalbzeug 2 abgebildet, das in einem einen Formkörper bildenden Einbettrohling 4, auch alternativ als "Blank" bezeichnet, eingebettet ist. In diesem Zusammenhang sei der guten Vollständigkeit halber noch angemerkt, dass im Gegensatz zu den beispielhaften Abbildungen in den Figuren erfindungsgemäß eine Mehrzahl von Knochenhalbzeugen 2 oder dergleichen prothetischen Elementen in den Einbettrohling 4 eingebracht sind Das Knochenhalbzeug 2 lässt sich auch allgemein als prothetisches Element bezeichnen und kann im vorliegenden Ausführungsbeispiel insbesondere autogene, isogene, xenogene oder allogene Knochenstücke aufweisen. Es ist aber auch denkbar, alternativ oder ggf. zusätzlich geeignetes künstliches Material für das Knochenhalbzeug 2 zu verwenden. Der Einbettrohling 4 weist ein Substrat 4a auf, das aus einem mit einem Werkzeug bearbeitbaren Material besteht und das Knochenhalbzeug 2 innerhalb des Einbettrohlings 4 fixiert.

Um das Knochenhalbzeug 2 in den Einbettrohling 4 zu verbringen, befindet sich zunächst das Material des Substrates 4a in einem fließfähigen Zustand in dem es mit dem Knochenhalbzeug 2 verbunden bzw. das Knochenhalbzeug 2 in das Substrat 4a eingebracht wird. Hierzu wird vorzugsweise das Knochenhalbzeug 2 in eine nicht dargestellte Form gelegt, in die anschließend das Substrat 4a in flüssiger Form eingefüllt wird. Zur Fixierung des Knochenhalbzeuges 2 innerhalb des Einbettrohlings 4 wird anschließend das Substrat 4a in einen festen Zustand überführt.

Hierzu ist für das Substrat 4a ein Material zu wählen, das in Abhängigkeit von der Temperatur seinen Aggregatzustand ändert, indem es sich bei einer höheren Temperatur im fließfähigen Zustand befindet und bei einer niedrigeren Temperatur einen festen Zustand einnimmt. Dabei kann die höhere Temperatur oberhalb der Zimmertemperatur liegen und die niedrigere Temperatur etwa im Wesentlichen Zimmertemperatur entsprechen. Alternativ ist es aber auch denkbar, dass die höhere Temperatur etwa im Wesentlichen Zimmertemperatur entspricht und die niedrigere Temperatur unterhalb der Zimmertemperatur liegt. Im letzteren Fall ist es auch denkbar, für das Substrat 4a ein Material zu wählen, das bei mindestens 0°C in den festen Zustand gelangt, wozu als Material für das Substrat 4a bevorzugt Wasser oder eine isotonische Kochsalzlösung verwendet werden kann, sodass dann der Einbettrohling 4 eine Art Eisblock bildet.

Erst wenn, wie bereits erwähnt, sich der Einbettrohling 4 mit dem darin eingebetteten Knochenhalbzeug 2 im festen Zustand befindet, ist das Knochenhalbzeug 2 innerhalb des Einbettrohlings 4 fixiert. Erst dann befindet sich der Einbettrohling 4 mit dem darin eingebetteten Knochenhalbzeug 2 in einem Zustand, der eine Aufbewahrung oder Weiterverarbeitung des Einbettrohlings 4 mit dem darin eingebetteten Knochenhalbzeug 2 ermöglicht.

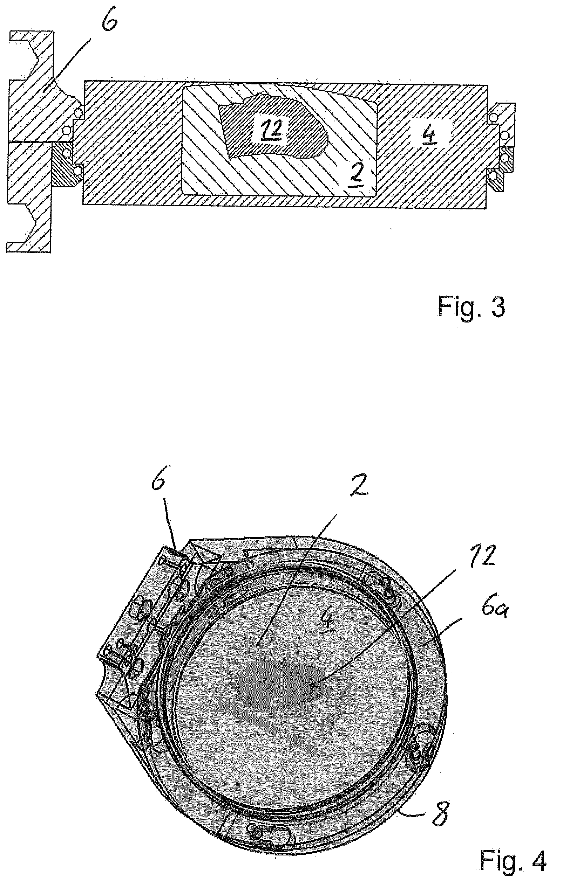

Im dargestellten Ausführungsbeispiel erhält der Einbettrohling 4 die Form eines scheibenförmigen Formkörpers, dessen umlaufender Rand 4b mit einem umlaufenden flanschartigen Vorsprung 4ba versehen ist, der in einem Abstand von den beiden Seiten bzw. Oberflächen des Einbettrohlings 4 endet, sodass der Rand 4b zwischen dem Vorsprung 4ba und der jeweils angrenzenden Oberfläche des Einbettrohlings 4 einen Hinterschnitt bzw. Absatz 4bb aufweist, wie insbesondere Fig. 1 in Verbindung mit Fig. 4 erkennen lässt.

Zur Weiterverarbeitung wird der Einbettrohling 4 in eine in den Figuren nicht abgebildete Bearbeitungseinrichtung eingespannt, deren Fixiereinrichtung 6 in den Fig. 2 bis 5 abschnittsweise abgebildet ist. Im dargestellten Ausführungsbeispiel weist die Fixiereinrichtung 6 einen nach innen umlaufenden flanschartigen Vorsprung 6a auf, an dessen Unterseite ein Spannring 8 angeordnet ist. Zwischen dem umlaufenden flanschartigen Vorsprung 6a der Fixiereinrichtung 6 und dem Spannring 8 wird der Einbettrohling 4 mit dem an seinem umlaufenden Rand 4b ausgebildeten umlaufenden flanschartigen Vorsprung 4ba eingespannt, wie beispielsweise Fig. 2 erkennen lässt.

Des Weiteren sind im dargestellten Ausführungsbeispiel sowohl innerhalb des umlaufenden flanschartigen Vorsprungs 6a der Fixiereinrichtung 6 als auch innerhalb des Spannringes 8 Kühlbohrungen 10 ausgebildet, wie beispielsweise Fig. 2 erkennen lässt. Diese Kühlbohrungen 10 sind Teil einer im Übrigen nicht dargestellten Temperaturhalteeinrichtung zum Halten der Temperatur des Einbettrohlings 4 auf die zuvor erwähnte untere Temperatur, insbesondere wenn das Substrat 4a aus einem Material besteht, das in dem zuvor genannten Temperaturbereich seinen Aggregatzustand ändert und sich bei der unteren Temperatur verfestigt. Damit wird gewährleistet, dass der Einbettrohling 4 mit dem darin eingebetteten Knochenhalbzeug 2 im festen Zustand verbleibt, was für eine nachfolgende Bearbeitung erforderlich ist.

Für die anschließende Bearbeitung des Einbettrohlings 4 und insbesondere des darin eingebetteten Knochenhalbzeugs 2 wird eine in den Figuren nicht abgebildete Erfassungseinrichtung verwendet, in die beispielsweise der Einbettrohling 4 angeordnet oder gelegt werden kann, um die exakte Position sowie bedarfsweise zusätzlich die jeweilige Qualität und auch die Dichte in demjenigen Bereich des Knochenhalbzeuges 2 zu ermitteln bzw. zu lokalisieren, aus dem dann ein gewünschtes Knochenprodukt herausgearbeitet werden soll, wie es beispielhaft in Figuren 3 und 4 skizziert und mit dem Bezugszeichen "12" bezeichnet ist. Hierfür sollte bevorzugt der Einbettrohling 4 auch mit einer exakten Nullpunktreferenzierung versehen sein, die zweckmäßigerweise entsprechend definierte Marker aufweist, wobei eine solche Nullpunktreferenzierung in den Figuren nicht abgebildet ist. Anschließend können dann die Daten des so erfassten Knochenproduktes 12 in eine CAM-Software eingeladen werden, um das herauszuarbeitende Knochenprodukt 12 innerhalb des Knochenhalbzeuges 2 und des dieses aufnehmenden Einbettrohlings 4 an der gewünschten Position zu lokalisieren.

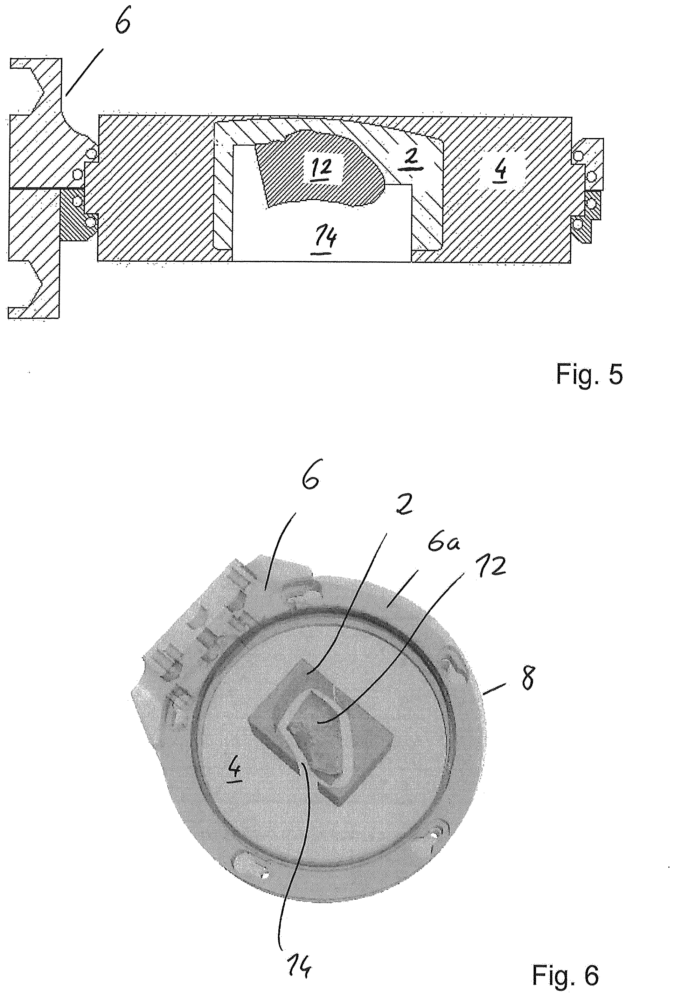

Danach wird das so identifizierte gewünschte Knochenprodukt 12 aus dem Knochenhalbzeug 2 mithilfe eines in den Figuren nicht abgebildeten Werkzeuges der Bearbeitungseinrichtung herausgetrennt. Um die Eigenschaften des gewünschten Knochenproduktes 12 sowie auch des zugrunde liegenden Knochenhalbzeugs 2 während des Bearbeitungsprozesses tolerabel zu erhalten bzw. diese nicht negativ zu beeinflussen, lässt sich auch während des Bearbeitungsprozesses die erwähnte, in den Figuren nicht abgebildete Erfassungseinrichtung entsprechend einsetzen oder kann ergänzend eine Messeinrichtung beispielsweise über Temperatur- und Drucksensoren implementiert werden. Hierzu zeigt Fig. 5 das Zwischenergebnis eines ersten Bearbeitungsschrittes, wonach in der Ansicht von Fig. 5 bereits unterhalb des Knochenproduktes 12 aus dem Knochenhalbzeug 2 und dem Einbettrohling 4 ein Bereich herausgearbeitet bzw. herausgetrennt worden ist, wodurch in diesem Bereich nun eine Aussparung 14 entstanden ist. Über die genannte Erfassungseinrichtung oder eine weitere Messapparatur wie beispielsweise ein bildgebendes Abtastsystem wird anschließend ein Soll-Ist-Vergleich in Bezug auf die gewünschte Formgebung des Knochenproduktes 12 durchgeführt und qualitätssichernd protokolliert.

In Fig. 6 ist schematisch im Wesentlichen das Endergebnis der Bearbeitung abgebildet, wonach das gewünschte Knochenprodukt 12 im Wesentlichen vollständig von der Aussparung 14 umgeben wird und somit sich bereits in einem vom Knochenhalbzeug 2 losgelösten Zustand befindet, sodass das so herausgearbeitete Knochenprodukt 12 nur noch entnommen werden muss und an der gewünschten Stelle im menschlichen oder tierischen Körper einzusetzen ist.

## Patentansprüche

1. Anordnung aus einer Mehrzahl von in einen menschlichen oder tierischen Körper einsetzbaren, natürlichen und/oder künstlichen prothetischen Elementen (2, 12), wie beispielsweise autogenen, isogenen, xenogenen oder allogenen Knochenstücken,
mit einem Formkörper (4), der ein Substrat (4a) aufweist, wobei das Substrat (4a) die prothetischen Elemente (2, 12) abschnittsweise oder im Wesentlichen vollständig enthält und aus einem mit einem Werkzeug bearbeitbaren Material besteht,
wobei das Material des Substrates (4a) von einem fließfähigen Zustand, in dem das Substrat (4a) mit den prothetischen Elementen (2, 12) verbindbar ist oder die prothetischen Elemente (2, 12) in das Substrat (4a) einbringbar sind, in einen festen Zustand überführbar ist,
**dadurch gekennzeichnet, dass** das Material des Substrates (4a) sich im fließfähigen Zustand befindet, wenn die auf das Material einwirkende Temperatur einen ersten Temperaturwert hat, und einen festen Zustand einnimmt, wenn die auf das Material des Substrates (4a) einwirkende Temperatur einen unterhalb des ersten Temperaturwertes liegenden zweiten Temperaturwert hat.

2. Anordnung nach Anspruch 1, **dadurch gekennzeichnet, dass** der erste Temperaturwert oberhalb der Zimmertemperatur liegt und der zweite Temperaturwert im Wesentlichen Zimmertemperatur entspricht.

3. Anordnung nach Anspruch 1, **dadurch gekennzeichnet, dass** der erste Temperaturwert im Wesentlichen Zimmertemperatur entspricht und der zweite Temperaturwert unterhalb der Zimmertemperatur liegt, wobei vorzugsweise der zweite Temperaturwert unter 0°C liegt und bevorzugt das Material des Substrates (4a) Wasser oder isotonische Kochsalzlösung ist.

4. Anordnung nach einem der vorangegangenen Ansprüche, **gekennzeichnet durch** eine Temperaturhalteeinrichtung (10), die ausgebildet ist, den Formkörper (4) auf den zweiten Temperaturwert zu halten.

5. Anordnung nach Anspruch 4, **dadurch gekennzeichnet, dass** der zweite Temperaturwert unter 0°C liegt und die Temperaturhalteeinrichtung (10) Kühlmittel aufweist.

6. Verfahren zum Einbringen einer Mehrzahl von in einen menschlichen oder tierischen Körper einsetzbaren, natürlichen und/oder künstlichen prothetischen Elementen (2, 12), wie beispielsweise autogenen, isogenen, xenogenen oder allogenen Knochenstücken, in eine Anordnung, die einen Formkörper (4) und ein Substrat (4a) aufweist, wobei das Substrat dazu eingerichtet ist, die prothetischen Elemente (2, 12) abschnittsweise oder im Wesentlichen vollständig zu enthalten,
**dadurch gekennzeichnet, dass**
I) die auf das Material des Substrates (4a) einwirkende Temperatur auf einen ersten Temperaturwert gebracht wird, bei dem sich das Material im fließfähigen Zustand befindet,
II) die prothetischen Elemente (2, 12) in das Substrat (4a) eingebracht werden und
III) anschließend die auf das Material des Substrates (4a) einwirkende Temperatur auf einen unterhalb des ersten Temperaturwertes liegenden zweiten Temperaturwert gebracht wird, bei dem das Material einen festen Zustand einnimmt,
wobei bevorzugt im Schritt II die prothetischen Elemente (2, 12) in eine Form gelegt werden und anschließend das Substrat (4a) im fließfähigen Zustand in die Form eingefüllt wird.

7. Vorrichtung zur Bearbeitung von in einen menschlichen oder tierischen Körper einsetzbaren, natürlichen und/oder künstlichen prothetischen Elementen (2, 12), wie beispielsweise autogenen, isogenen, xenogenen oder allogenen Knochenstücken, unter Verwendung einer Anordnung nach mindestens einem der Ansprüche 1 bis 5,
wobei die Vorrichtung eine Erfassungseinrichtung zur Erfassung mindestens der Position der prothetischen Elemente (2, 12) innerhalb des Formkörpers (4) und eine Bearbeitungsvorrichtung aufweist, und wobei die Bearbeitungsvorrichtung ein Werkzeug zum abschnittsweisen oder vollständigen Heraustrennen von prothetischen Elementen (12) aus dem Formkörper (4) aufweist.

8. Vorrichtung nach Anspruch 7, **dadurch gekennzeichnet, dass** die Erfassungseinrichtung zusätzlich zur Erfassung der Temperatur und/oder der Zusammensetzung und/oder der Qualität der prothetischen Elemente (2, 12) vorgesehen ist und/oder ein Röntgen-Gerät und/oder ein Computertomografie-Gerät und/oder mindestens einen Temperatursensor aufweist und/oder eine Fixiereinrichtung (6, 8) zum Einspannen des Formkörpers (4) vorgesehen ist.

9. Vorrichtung nach Anspruch 7 oder 8, **dadurch gekennzeichnet, dass** zur Herstellung einer Anordnung nach Anspruch 2 eine Form vorgesehen ist, in der die prothetischen Elemente (2, 12) anordenbar und in die das Substrat im fließfähigen Zustand einfüllbar ist.

10. Vorrichtung nach mindestens einem der Ansprüche 7 bis 9, **dadurch gekennzeichnet, dass** zur Herstellung einer Anordnung nach Anspruch 5 eine Temperaturhalteeinrichtung (10) zum Halten der Temperatur des Formkörpers (4) auf den zweiten Temperaturwert vorgesehen ist, wobei bevorzugt die Temperaturhalteeinrichtung (10) bei einer Anordnung, bei der der erste Temperaturwert im Wesentlichen Zimmertemperatur entspricht und der zweite Temperaturwert unterhalb der Zimmertemperatur liegt, bevorzugt unter 0°C, liegt, eine Kühleinrichtung aufweist, die den Formkörper auf einen zweiten Temperaturwert unterhalb der Zimmertemperatur, vorzugsweise unter 0°C, kühlt.

11. Verfahren zur Bearbeitung von in einen menschlichen oder tierischen Körper einsetzbaren, natürlichen und/oder künstlichen prothetischen Elementen (2, 12), wie beispielsweise autogenen, isogenen, xenogenen oder allogenen Knochenstücken, unter Verwendung einer Anordnung nach mindestens einem der Ansprüche 1 bis 5 und einer Vorrichtung nach mindestens einem der Ansprüche 7 bis 10,
wobei
A) mithilfe der Erfassungseinrichtung mindestens die Position der prothetischen Elemente (2, 12) innerhalb des Formkörpers (4) ermittelt wird,
B) in Abhängigkeit von mindestens der von der Erfassungseinrichtung erfassten Position der im Formkörper (4) enthaltenen prothetischen Elemente (2, 12) das Werkzeug der Bearbeitungseinrichtung in den Formkörper (4) zu den prothetischen Elementen (2, 12) mit der von der Erfassungseinrichtung erfassten Position geführt wird und
C) das prothetische Element (12) oder die prothetischen Elemente mit der von der Erfassungseinrichtung erfassten Position mithilfe des Werkzeugs der Bearbeitungseinrichtung aus dem Formkörper (4) herausgetrennt wird bzw. werden.

12. Verfahren nach Anspruch 11 unter Verwendung einer Fixiereinrichtung (6, 8) zum Einspannen des Formkörpers (4),
**dadurch gekennzeichnet, dass** vor Schritt B der Formkörper (4) in der Fixiereinrichtung (6, 8) eingespannt wird.

13. Verfahren nach Anspruch 11 oder 12, **dadurch gekennzeichnet, dass** in Schritt A mithilfe der Erfassungseinrichtung des Weiteren die Dichte der prothetischen Elemente (2, 12) erfasst wird.

## Claims

1. Arrangement of a plurality of natural and/or artificial prosthetic elements (2, 12) that can be inserted into a human or animal body, such as autogenous, isogeneic, xenogeneic or allogeneic bone pieces,
comprising a shaped body (4) that has a substrate (4a), the substrate (4a) containing the prosthetic elements (2, 12) in portions or substantially completely and consisting of a material that is machinable with a tool,
wherein the material of the substrate (4a) can be converted from a free-flowing state, in which the substrate (4a) can be connected to the prosthetic elements (2, 12) or the prosthetic elements (2, 12) can be introduced into the substrate (4a), into a solid state, **characterised in that** the material of the substrate (4a) is in the free-flowing state when the temperature acting on the material has a first temperature value and assumes a solid state when the temperature acting on the material of the substrate (4a) has a second temperature value that is below the first temperature value.

2. Arrangement according to claim 1, **characterised in that** the first temperature value is above room temperature and the second temperature substantially corresponds to room temperature.

3. Arrangement according to claim 1, **characterised in that** the first temperature value substantially corresponds to room temperature and the second temperature value is below room temperature, wherein preferably the second temperature is below 0°C and the material of the substrate (4a) is preferably water or isotonic saline solution.

4. Arrangement according to one of the preceding claims, **characterised by** a temperature-maintaining device (10) designed to maintain the shaped body (4) at the second temperature value.

5. Arrangement according to claim 4, **characterised in that** the second temperature value is below 0°C and the temperature-maintaining device (10) comprises coolant.

6. Method for introducing a plurality of natural and/or artificial prosthetic elements (2, 12) that can be inserted into a human or animal body, such as autogenous, isogeneic, xenogeneic or allogeneic bone pieces, into an arrangement having a shaped body (4) and a substrate (4a), wherein the substrate is configured to contain the prosthetic elements (2, 12) in portions or substantially completely,
**characterised in that**
I) the temperature acting on the material of the substrate (4a) is brought to a first temperature value at which the material is in the free-flowing state,
II) the prosthetic elements (2, 12) are introduced into the substrate (4a), and
III) then the temperature acting on the material of the substrate (4a) is brought to a second temperature value that is below the first temperature value and at which the material assumes a solid state,
wherein preferably, in step II, the prosthetic elements (2, 12) are placed in a mould and then the substrate (4a) is filled into the mould in the free-flowing state.

7. Apparatus for machining natural and/or artificial prosthetic elements (2, 12) that can be inserted into a human or animal body, such as autogenous, isogeneic, xenogeneic or allogeneic bone pieces, using an arrangement according to at least one of claims 1 to 5, wherein the apparatus has a detection device for detecting at least the position of the prosthetic elements (2, 12) inside the shaped body (4) and a machining apparatus, and wherein the machining apparatus has a tool for removing prosthetic elements (12) from the shaped body (4) in portions or completely.

8. Apparatus according to claim 7, **characterised in that** the detection device is additionally provided for detecting the temperature and/or composition and/or quality of the prosthetic elements (2, 12) and/or has an X-ray device and/or a computed tomography device and/or at least one temperature sensor and/or a fixing device (6, 8) is provided for clamping the shaped body (4).

9. Apparatus according to claim 7 or 8, **characterised in that** in order to produce an arrangement according to claim 2, a mould is provided in which the prosthetic elements (2, 12) can be arranged and into which the substrate can be filled in the free-flowing state.

10. Apparatus according to at least one of claims 7 to 9, **characterised in that** in order to produce an arrangement according to claim 5, a temperature-maintaining device (10) for maintaining the temperature of the shaped body (4) at the second temperature value is provided, wherein the temperature-maintaining device (10), in an arrangement in which the first temperature value substantially corresponds to room temperature and the second temperature value is below room temperature, preferably below 0°C, preferably has a cooling device which cools the shaped body to a second temperature value below room temperature, with preference below 0°C.

11. Method for machining natural and/or artificial prosthetic elements (2, 12) that can be inserted into a human or animal body, such as autogenous, isogeneic, xenogeneic or allogeneic bone pieces, using an arrangement according to at least one of claims 1 to 5 and an apparatus according to at least one of claims 7 to 10,
wherein
A) the detection device is used to determine at least the position of the prosthetic elements (2, 12) within the shaped body (4),
B) depending on at least the position, detected by the detection device, of the prosthetic elements (2, 12) contained in the shaped body (4), the tool of the machining device is guided in the shaped body (4) to the prosthetic elements (2, 12) having the position detected by the detection device, and
C) the prosthetic element (12) or the prosthetic elements having the position detected by the detection device is or are removed from the shaped body (4) using the tool of the machining device.

12. Method according to claim 11 using a fixing device (6, 8) to clamp the shaped body (4),
**characterised in that** prior to step B, the shaped body (4) is clamped in the fixing device (6, 8).

13. Method according to claim 11 or 12, **characterised in that** in step A, the detection device is furthermore used to detect the density of the prosthetic elements (2, 12).

## Revendications

1. Ensemble composé d'une multitude d'éléments prothétiques (2, 12) naturels et/ou artificiels pouvant être insérés dans un corps humain ou animal, tels que des morceaux d'os autogènes, isogènes, xénogènes ou allogènes,
avec un corps moulé (4), qui présente un substrat (4a), dans lequel le substrat (4a) contient par endroits ou sensiblement en totalité les éléments prothétiques (2, 12) et est constitué d'un matériau pouvant être usiné avec un outil,
dans lequel le matériau du substrat (4a)
peut être transféré d'un état coulant, dans lequel le substrat (4a) peut être relié aux éléments prothétiques (2, 12) ou dans lequel les éléments prothétiques (2, 12) peuvent être introduits dans le substrat (4a), à un état solide,
**caractérisé en ce que** le matériau du substrat (4a)
se trouve dans l'état coulant lorsque la température agissant sur le matériau a une première valeur de température, et adopte un état solide lorsque la température agissant sur le matériau du substrat (4a) a une deuxième valeur de température inférieure à la première valeur de température.

2. Ensemble selon la revendication 1, **caractérisé en ce que** la première valeur de température est supérieure à la température ambiante et la deuxième valeur de température correspond sensiblement à la température ambiante.

3. Ensemble selon la revendication 1, **caractérisé en ce que** la première valeur de température correspond sensiblement à la température ambiante et la deuxième valeur de température est inférieure à la température ambiante, dans lequel de préférence la deuxième valeur de température est inférieure à 0 °C et de manière préférée le matériau du substrat (4a) est de l'eau ou une solution saline isotonique.

4. Ensemble selon l'une quelconque des revendications précédentes, **caractérisé par** un système de maintien de température (10), qui est réalisé pour maintenir le corps moulé (4) à la deuxième valeur de température.

5. Ensemble selon la revendication 4, **caractérisé en ce que** la deuxième valeur de température est inférieure à 0 °C et le système de maintien de température (10) présente un liquide de refroidissement.

6. Procédé d'introduction d'une multitude d'éléments prothétiques naturels et/ou artificiels (2, 12) pouvant être insérés dans un corps humain ou animal, tels que des morceaux d'os autogènes, isogènes, xénogènes ou allogènes, dans un ensemble, qui présente un corps moulé (4) et un substrat (4a), dans lequel le substrat est mis au point pour contenir par endroits ou sensiblement en totalité les éléments prothétiques (2, 12),
**caractérisé en ce que**
I) la température agissant sur le matériau du substrat (4a) est amenée à une première valeur de température à laquelle le matériau se trouve dans l'état coulant,
II) les éléments prothétiques (2, 12) sont introduits dans le substrat (4a) et
III) puis la température agissant sur le matériau du substrat (4a) est amenée à une deuxième valeur de température inférieure à la première valeur de température, à laquelle le matériau adopte un état solide,
dans lequel, de manière préférée à l'étape II, les éléments prothétiques (2, 12) sont placés dans un moule puis le substrat (4a) est transvasé dans le moule dans l'état coulant.

7. Dispositif d'usinage d'éléments prothétiques (2, 12) naturels et/ou artificiels pouvant être insérés dans un corps humain ou animal, tels que des morceaux d'os autogènes, isogènes, xénogènes ou allogènes, en utilisant un ensemble selon au moins l'une quelconque des revendications 1 à 5,
dans lequel le dispositif présente un système de détection destiné à détecter au moins la position des éléments prothétiques (2, 12) à l'intérieur du corps moulé (4) et un dispositif d'usinage, et dans lequel le dispositif d'usinage présente un outil destiné à séparer en partie ou en totalité des éléments prothétiques (12) du corps moulé (4).

8. Dispositif selon la revendication 7, **caractérisé en ce que** le système de détection est prévu en supplément pour détecter la température et/ou la composition et/ou la qualité des éléments prothétiques (2, 12) et/ou présente un appareil à rayons X et/ou un appareil de tomodensitométrie et/ou au moins un capteur de température et/ou un système de fixation (6, 8) destiné à enserrer le corps moulé (4).

9. Dispositif selon la revendication 7 ou 8, **caractérisé en ce que**, pour la réalisation d'un ensemble selon la revendication 2, un moule est prévu, dans lequel les éléments prothétiques (2, 12) peuvent être disposés et dans lequel le substrat peut être transvasé dans l'état coulant.

10. Dispositif selon au moins l'une des revendications 7 à 9, **caractérisé en ce que**, pour la réalisation d'un ensemble selon la revendication 5, un système de maintien de température (10) est prévu pour maintenir la température du corps moulé (4) à la deuxième valeur de température, dans lequel de manière préférée le système de maintien de température (10) présente un système de refroidissement, qui refroidit le corps moulé à une deuxième valeur de température inférieure à la température ambiante, de préférence inférieure à 0 °C, pour un ensemble, pour lequel la première valeur de température correspond sensiblement à la température ambiante et la deuxième valeur de température est inférieure à la température ambiante, de manière préférée est inférieure à 0 °C.

11. Procédé d'usinage d'éléments prothétiques (2, 12) naturels et/ou artificiels pouvant être insérés dans un corps humain ou animal, tels que des morceaux d'os autogènes, isogènes, xénogènes ou allogènes, en utilisant un ensemble selon au moins l'une des revendications 1 à 5 et un dispositif selon au moins l'une quelconque des revendications 7 à 10,
dans lequel
A) au moins la position des éléments prothétiques (2, 12) à l'intérieur du corps moulé (4) est déterminée à l'aide du système de détection,
B) en fonction d'au moins la position des éléments prothétiques (2, 12) contenus dans le corps moulé (4), détectée par le système de détection, l'outil du système d'usinage est guidé dans le corps moulé (4) vers les éléments prothétiques (2, 12) avec la position détectée par le système de détection et
C) l'élément prothétique (12) ou les éléments prothétiques sont séparés du corps moulé (4) avec la position détectée par le système de détection à l'aide de l'outil du système d'usinage.

12. Procédé selon la revendication 11 utilisant un système de fixation (6, 8) destiné à enserrer le corps moulé (4),
**caractérisé en ce que** le corps moulé (4) est enserré dans le système de fixation (6, 8) avant l'étape B.

13. Procédé selon la revendication 11 ou 12, **caractérisé en ce que**, à l'étape A,
la densité des éléments prothétiques (2, 12) est détectée par ailleurs à l'aide du système de détection.
